# EUROPÄISCHE PATENTSCHRIFT

(11) **EP 0 557 825 B1**
(45) Veröffentlichungstag und Bekanntmachung des Hinweises auf die Patenterteilung: **13.05.1998**
(21) Anmeldenummer: 93102232.1
(22) Anmeldetag: 12.02.1993
(51) Int. Cl.: A61K 7/50, A61K 7/48, A61K 7/00

(54) **Bade- und Duschzusätze mit vesikelbildenden Eigenschaften, ihre Herstellung und Verwendung**
Bath and shower additives with vesicle forming properties, preparation and use thereof
Additifs pour bain et douche formant des vésicules, leur préparation et application

(30) Priorität: 24.02.1992 DE 4205548
(43) Veröffentlichungstag der Anmeldung: 01.09.1993
(73) Patentinhaber: Merz & Co. GmbH & Co., D-60318 Frankfurt (DE)
(72) Erfinder: Nürnberg, E., Prof.Dr., W-8521 Uttenreuth (DE); Gassenmeier, Thomas, W-8500 Nürnberg 30 (DE); Beutler, Rolf, D., Dr., W-6128 Höchst/Odenwald (DE); Ebinger, Jürgen, W-6274 Hünstetten 8 (DE)
(74) Vertreter: Beil, Hans Chr., Dr.

(56) Entgegenhaltungen:
- DE-A- 4 021 083
- DEUTSCHE APOTHEKER ZEITUNG, Bd.133, Nr.41, 14. Oktober 1993 Seiten 43 - 50 E. NÜRNBERG 'Oleobalneologika. Verteilungszustände lipophiler Stoffe in Badeflotten.'

## Beschreibung

Die vorliegende Erfindung betrifft neue ölhaltige Bade- und Duschzusätze, welche eine Kombination aus öllöslichen Tensiden sowie vesikelbildenden Lipiden und gegebenenfalls weitere Wirkstoffe enthalten. Diese Zusammensetzungen bilden spontan beim Eingießen und/oder Verteilen in Wasser dispergierte Vesikel. Sie eignen sich insbesondere zur Herstellung von Zusätzen für Voll-, Teil- und Duschbäder.

Für balneologische und/oder kosmetische Zwecke werden verschiedene Zubereitungen mit einem Gehalt an oberflächenaktiven Stoffen angewendet. Diese enthalten beispielsweise wassermischbare oder wasserdispergierbare Tenside und gegebenenfalls Parfümstoffe sowie zusätzliche Wirkstoffe in Form von ätherischen Ölen, pflanzlichen Extrakten, Vitaminen sowie weiteren Wirkstoffen. Grundlagen dieser Präparate sind ionische oder nichtionische Tensidsubstanzen sowie die genannten Wirkstoffe, die nach Eingabe in ein Badewasser eine gleichmäßige Verteilung der Komponenten ermöglichen. Diese Präparate sind entweder klar oder sie weisen eine mehr oder weniger ausgeprägte Opaleszenz auf. Die Badeflotte erscheint klar oder zeigt eine gewisse Trübung.

Außer diesen Präparaten sind auch ölhaltige balneologische und/oder kosmetische Zubereitungen bekannt. Diese Produkte unterscheiden sich von den oben beschriebenen dadurch, daß sie neben einem Gehalt an oberflächenaktiven Tensiden einen relativ hohen Anteil fetter Öle oder anderer lipophiler flüssiger Komponenten enthalten. Im Gegensatz zu den ölfreien Präparationen ergeben diese stets trübe Badeflotten, in denen zunächst die lipophilen Bestandteile relativ gleichmäßig verteilt werden. Im Verlauf von ca. 10 bis 30 Minuten tritt jedoch eine zunehmende Flotation an die Oberfläche ein. Umfang und Geschwindigkeit des Aufrahmungsvorgangs sind von verschiedenen Faktoren, wie beispielsweise Art und Menge der enthaltenen Tenside bzw. Dispersionsmittel, Art und Menge der lipophilen Stoffe und der Temperatur des Badewassers abhängig.

Die DE-A 4021083 beschreibt balneologische Zusammensetzungen, enthaltend Phosphatidylcholin und eine Ölkomponente sowie zwingend Alkohol, einen Stabilisator und einen Wirkstoff. Hierdurch sollen liposomen-stabile Produkte erhalten werden, die alle Vorteile des Ölbades mit Liposomen aufweisen, jedoch die durch bestimmte Emulgatoren hervorgerufenen Nachteile umgehen sollen.

Außer den genannten Präparaten sind auch solche bekannt, die nur ein fettes Öl - gegebenenfalls unter Zusatz weiterer öliger Komponenten - und eventuell weitere aktive Wirkstoffe wie etherische Öle u.ä. enthalten. Da diese Präparate ohne oberflächenaktive Stoffe nach Zugabe zum Badewasser eine unbefriedigende Dispersität der lipophilen Bestandteile ergeben und unmittelbar nach dem Zusatz auf der Oberfläche der Badeflotte eine mehr oder weniger gleichmäßige Schicht bilden, sind sie für die balneologische Anwendung eher ungeeignet.

Zur Erzielung eines befriedigenden balneologischen und/oder kosmetischen Effektes sind Dispersionsmittel bzw. oberflächenaktive Substanzen zur dispersen Verteilung des Öls im Wasser erforderlich. Dadurch ist eine gleichmäßige Verteilung der Ölphase auf der Hautoberfläche gewährleistet. Darüberhinaus sollte die Penetration der in den Präparationen enthaltenen Wirkstoffe in die oberen Hautschichten gefördert werden, um so eine Wirkungsverstärkung zu erzielen.

Dies ist jedoch mit den bisher bekannten Zusammensetzungen aufgrund fehlender vesikulärer Eigenschaften in der Badeflotte nicht möglich.

Aufgabe vorliegender Erfindung ist es daher, einen balneologischen Zusatz bereitzustellen, durch welchen ohne Einsatz aufwendiger Verfahren bei der Anwendung im Bad/Dusche Vesikel vorliegen, wodurch der Zusatz nicht nur oberflächenaktiv wirkt, sondern auch die inkorporierten Wirkstoffe und Komponenten protrahiert freigesetzt werden können und somit der balneologische bzw. kosmetische Effekt erheblich verstärkt werden kann. Hierbei soll auf Komponenten wie Alkohol und Stabilisatoren (wie in der DE-A 4021083 bechrieben ) verzichtet werden.

Diese Aufgabe wird erfindungsgemäß dadurch gelöst, daß Bade- bzw. Duschzusätze bereitgestellt werden, welche als Basis fette Öle und/oder andere entsprechende apolare Substanzen, gegebenenfalls wirksame Komponenten, und darüberhinaus eine Kombination aus öllöslichen Tensiden sowie vesikelbildenden Lipiden enthalten.

Solche Zusammensetzungen zeichnen sich überraschenderweise dadurch aus, daß sie bei der Anwendung in und/oder mit Wasser spontan Vesikel bilden, obgleich bekannt ist, daß zur Herstellung von Vesikeln spezielle Methoden und Apparaturen erforderlich sind. Bei allen Verfahren ist kennzeichnend, daß zunächst lamellare Einheiten gebildet werden müssen, die unter Anwendung energiereicher Verfahren, wie beispielsweise Ultraschall, Hochdruckdüsen oder schnellaufender Rühraggregate in eine globuläre Form mit geringer Partikelgröße überführt werden müssen.

Um so überraschender war es, daß mit den erfindungsgemäßen Zusammensetzungen bei der Anwendung in oder mit Wasser Vesikel auftreten, obwohl, wie oben erläutert, zum einen Vesikel normalerweise erst durch Anwendung hoher externer Energie gebildet werden und zum anderen bei Inkorporation der vesikelbildenden Bestandteile in Ölbäder durch deren hohen Gehalt an fetten Ölen eigentlich aufgelöst werden sollten, so daß das Vorliegen von Vesikeln in Wasser verhindert sein müßte. Die erfindungsgemäß erhaltenen Vesikel stellen globuläre Einheiten aus konzentrisch aufgebauten Bilayerschichten dar. In Abhängigkeit von der Größe und der möglicherweise übereinanderliegenden größeren Anzahl von Bilayerschichten werden unilamellare und multilamellare bzw. oligolamellare Vesikel unterschieden. Diese Tensidassoziate zeichnen sich durch eine bemerkenswerte Stabilität und durch ihre Anisotropie aus. Sie tritt häufig bei polarisationsmikroskopischer Betrachtung in Erscheinung. Sie sind - im Falle der multilamellaren Vesikel - daher bereits im Polarisationsmikroskop deutlich erkennbar.

Im Unterschied zu bisher hergestellten Vesikeln ist erfindungsgemäß jedoch kein stabilisierender Hilfsstoff, wie z.B. Cholestrol, erforderlich.

Mit den neuen Zusätzen ist es somit möglich, den balneologischen und/oder kosmetischen Effekt solcher Zusammensetzungen erheblich zu verstärken, da die Wirkstoffe über die Liposomen bzw. Vesikel nicht nur auf der Haut wirken, sondern langanhaltend in der Haut wirken können, da die Ölkomponente als Wirkungsträger und Überträger als Depot für die Wirkstoffe dient, die protrahiert freigesetzt werden können. Somit ist mit den erfindungsgemäßen Ölbädern eine über den bisherigen Kurzzeitpflegeeffekt hinausgehende Wirkung erzielbar, da Liposomen und Vesikel bekanntlich die Epidermis günstig beeinflussen im Hinblick auf die Einlagerung von Lipiden und Wirkstoffen und u.a. das Hydratationsvermögen der Haut günstig beeinflussen.

Die erfindungsgemäßen Ölbäder enthalten übliche Ölkomponenten und vorzugsweise Erdnuß-, Sesam-, Sonnenblumen-, Soja-, Jojoba-, Ricinus-, Mandel-, Oliven- Nerz- und Weizenkeimöl oder deren Mischungen.

Weitere geeignete Komponenten der Ölphase können apolare Substanzen, wie z.B. Paraffinkohlenwasserstoffe, Ester höherer Alkohole und höherer Fettsäuren (sog. Wachsester) sowie höhere Fettalkohole sein. Bevorzugte Komponenten sind:
Paraffinöl
Isopropylmyristat
Isopropylpalmitat
mittelkettige Triglyceride
Propylenglycolester von mittelkettigen Fettsäuren
2-Octyldodecanol
Laurinsäurehexylester
Isooctylstearat
Capryl/Caprinsäuretriglycerid u.a.
Cetylpalmitat.

Diese können einzeln oder als Gemisch zusammen mit den obengenannten Ölkomponenten oder deren Mischungen vorliegen. Es handelt sich hierbei um für die Herstellung von Ölbädern bekannte Stoffe.
Insbesondere bevorzugt sind Soja-, Paraffin-, Jojobaöl, Isopropylmyristat, Isopropylpalmitat, Caprensäuretriglycerid oder deren Mischungen.

Als öllösliche oberflächenaktive Substanzen (Tenside) zur Dispergierung der Ölphase sind hierfür übliche Tenside verwendbar. Hierzu gehören öllösliche, in Wasser dispergierbare Substanzen mit einem HLB-Wert von 6 bis 13, vorzugsweise 7 bis 10. Insbesondere sind folgende Stoffe geeignet:
Polyoxyethylenlaurylether mit 1 bis 4 EO-Einheiten mittlerer Kettenlänge
Polyoxyethylen(5)Oleylether
Polyoxyethylen(7)Glycerylcocoat
Cocosfettsäurediethanolamid
Mono/Di/Tri/(alkyltetraglycolether)/o/Phosphosäureester
Polyoxyethylen-glyceroltrioleat
Polyoxyethylensorbitantristearat
Polyoxypropylen(15)Stearylether.

Insbesondere bevorzugt sind Polyoxyethylenlaurylether mit 1 bis 4 EO-Einheiten, Polyoxyethylen(7)Glycerolcocoat, Cocosfettsäurediethanolamid oder deren Mischungen.

Als vesikelbildende Lipide eignen sich hierfür bekannte Stoffe, insbesondere polyoxyethylierte Fettalkohole mit vorzugsweise 1 bis 4 EO, mit einem HLB-Wert von 2 bis 6, wobei der lipophile Rest bevorzugt aus dem Bereich von C₁₆- bis C₁₈-Fettalkoholen besteht.

Ferner sind auch analoge Verbindungen der entsprechenden Fettsäuren sowie Phospholipide geeignet. Hierzu zählen insbesondere Lecithin (Ei- oder Soja-Lecithin), Phosphatidylcholin, -serin oder -diethanolamin sowie deren Mischungen.

Weitere geeignete Komponenten zur Herstellung von Vesikeln oder Mischvesikeln sind Sphingolipide (z.B. Sphingosin, Ceramide, Cerebroside, Sphingomyelin), Cholesterol, quartäre Ammoniumverbindungen, Polyglycerolalkylether, Glucosyldialkylether, Saccharosediester, Schwefelsäureester höherer Alkohole, Kollagenhydrolysatester, Gallensäure und Poloxamere.

Diese Komponenten sind zur Herstellung von Liposomen sind bereits bekannt (vgl. DE 41 21 945.7). Dabei wurden die Vesikel jedoch erst durch aufwendige Verfahren hergestellt und in die gewünschte Zubereitung eingearbeitet, wobei zumeist Stabilisatoren erforderlich waren, was erfindungsgemäß nicht der Fall ist.

Wie erwähnt können die erfindungsgemäßen Zusammensetzungen darüberhinaus bevorzugt 0 bis 30%, insbesondere 5 bis 15%, wirksame Komponenten, wie z.B. etherische Öle, Wirkstoffextrakte und/oder Vitamine in üblichen Mengen enthalten. Hierzu zählen insbesondere Oleum menthae japonicum, Rosmarinöl, Lavendelöl, Menthol, ferner Jojobaöl, Lindenblüten-, Ringelblumen-, Aloe-Vera-Extrakt, Vitamin E, Avocadoöl, Efeublätterextrakt, Eukalyptusöl, Thymianöl, Phyto-Lipide u.a.. Dabei kann je nach beabsichtigtem Wirkungseffekt, wie z.B. Verbesserung der Hautstruktur, Pflege hochsensibler Haut, Erhöhung der Durchblutung, Entspannung u.ä., eine geeignete Kombination an Wirkstoffen zugesetzt werden. Gegebenenfalls können übliche Hilfsstoffe wie Verdickungsmittel, z.B. Aerosil, Aluminiumstearat, Magnesiumstearat oder analoge Substanzen und Gemische, je nach Konfektionierungsart in üblichen Mengen (z.B. 1-10%) hinzugesetzt werden.

Als besonders geeignete Kombinationen erwiesen sich solche, die 20% bis 90% Ölkomponente (fette Öle und/oder apolare Substanzen), 2 bis 50% öllösliche Tenside, 0,1 bis 20% vesikelbildende Lipide, gegebenenfalls 0 bis 30% wirksame Komponenten, enthalten.

Insbesondere zeigen Zusammensetzungen, welche 50 bis 70% einer oder mehrerer der genannten Ölkomponenten sowie 5 bis 35%, insbesondere 10 bis 20% öllösliches Tensid oder Gemische hiervon und 0,5 bis 5% Lipid, gegebenenfalls 5 bis 15% wirksame Komponenten enthalten, sehr gute vesikelbildende Eigenschaften.

Die erfindungsgemäßen Zusätze entfalten bei der Anwendung in Wasser einerseits einen Sofort-Effekt und andererseits eine Depotwirkung. Diese 2-Phasen-Wirkung führt aufgrund der vesikulären Struktur der erfindungsgemäßen Zusätze in Wasser zu einer erheblichen Steigerung und Verlängerung des beabsichtigten Effekts bzw. zum Auftreten eines kosmetischen Pflegeeffekts auf liposomaler Grundlage, ohne daß hier jedoch mehr Aufwendungen bezüglich der Anwendung des Präparates bzw. seiner Herstellung erforderlich wären.

Darüberhinaus liegt bei den erfindungsgemäßen Zusammensetzungen auch eine gleichmäßige Verteilung der Ölphase im Wasser vor. Zwar besitzen vesikelbildende Lipide an sich W/O-Emulgatoreigenschaften. Sie können jedoch normalerweise die Verteilung oder Dispergierung der im großen Überschuß vorhandenen Öle im Badewasser nicht hinreichend gewährleisten. Der erfindungsgemäße Zusatz an weniger lipophilen Tensiden gleicht dies aus. Aufgrund der erzielten gleichmäßigen Verteilung wird eine Vesikelbildung erst ermöglicht. Die erfindungsgemäßen Rezepturen sind demnach einerseits durch ihr hohes Emulgiervermögen der Ölphase in der Badeflotte und andererseits durch die spontane Vesikelbildung gekennzeichnet. Beide Komponenten sind somit für die Herstellung der erfindungsgemäßen Präparate wesentlich und führen zu transparenten Zusammensetzungen.

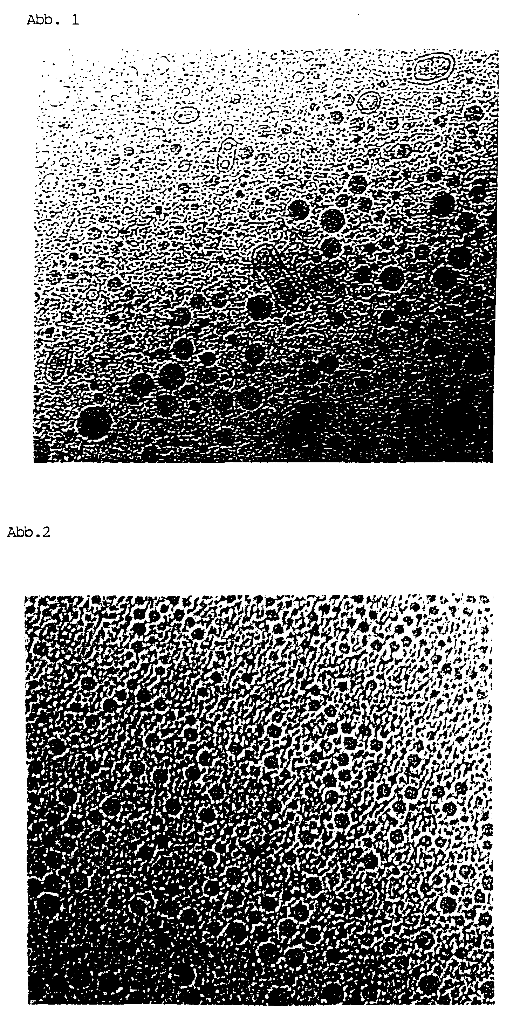

In Abbildung 1 ist das Vorliegen vesikulärer Bestandteile (Mehrfachkreise) in einer mit einem erfindungsgemäßen Produkt (gemäß Beispiel 1) hergestellten Badeflotte elektronenmikroskopisch dargestellt.

Als Vergleich zeigt Abbildung 2 die Struktur einer Badeflotte mit einer Zusammensetzung gemäß Stand der Technik (Ölbad + Tensid, ohne Lipid). Wie hieraus deutlich ersichtlich ist (nur Einfachkreise), liegen hier keine Liposomen vor.

Die erhöhte Effektivität der erfindungsgemäßen Produkte wurde in klinischen Versuchen belegt. Hierzu wurden 2 galenische Formulierungen gemäß vorliegender Erfindung an freiwilligen Probanden getestet.

### Test 1

Ein Produkt gemäß vorliegender Erfindung, enthaltend 70% Öl/apolare Substanzen, 20% Tensid, 3% Lecithin sowie Eukalyptusöl, Kiefernnadelöl und Thymianöl als aktive Bestandteile (Zusammensetzung gemäß Beispiel 1), wurde wie folgt getestet:

Es wurde untersucht, ob und wie stark sich durch das neue 2-Phasen-Bad besonderer Galenik (im Bad spontane Bildung vesikulärer Lipid-Partikel mit Aktivstoffen) die kutane Mikrozirkulation verbessern läßt.

Es wurden 10 hautgesunde freiwillige Probanden in die Studie mit einbezogen. Die Untersuchungen wurden in einer (üblicherweise vorgenommenen) Dosierung von 30 g Badekonzentrat auf 100 l Badewasser bei 37°C durchgeführt.

Zum objektiven Vergleich wurden auch entsprechende Messungen in reinem Wasser durchgeführt.

Zur Erfassung der kutanen Mikrozirkulation wurden folgende Meßmethoden herangezogen:
- Laser Doppler-Messung des Erythrozyten-Fluxes
- Transkutane Sauerstoffpartialdruckmessung
- Messung der Hauttemperatur

### Ergebnisse:

An der gesunden Haut läßt sich durch das erfindungsgemäße Produkt gegenüber dem reinen Wasserbad eine deutliche Steigerung der kutanen Sauerstoffversorgung um 212% erzielen. Der Erythrozytenfluß in den tieferen, dermalen Gefäßen und auch im Kapillarbereich ist um 66% gesteigert.

Die Verbesserung der kutanen Sauerstoffversorgung und Durchblutung hält mit 160% bzw. 33% über eine Stunde nach Badeende an. Dies kann durch den sogenannten Depoteffekt des 2-Phasen-Bades erklärt werden, der eine verlängerte und intensivere Wirkung der Aktivstoffe auf die menschliche Haut ermöglicht.

Die besondere galenische Komposition des erfindungsgemäßen Präparates mit dem Wirkkomplex aus Lipid-Partikeln ermöglicht ein Aufziehen der Wirkstoffe auf die Haut während des Badevorgangs (Sofortwirkung) und protrahierte Abgabe der Aktivstoffe an die Haut nach dem Bad (Depotwirkung).

Dabei kommt es zu einer deutlichen Anregung der kutanen Durchblutung, vor allem in den tieferen dermalen Gefäßen, und Verbesserung der epidermalen Sauerstoffversorgung. Dies trägt zu einer allgemeinen Aktivierung und Stärkung der körpereigenen Widerstandskraft bei.

### Test 2

Auf die oben beschriebene Weise wurde an 10 freiwilligen hautgesunden Probanden eine erfindungsgemäße Präparation, enthaltend 70% fettes Öl/apolare Substanzen, 10% öllösliche Tenside und 5% Lecithin sowie Rosmarinöl, Kiefernnadelöl, Efeublätterextrakt und Vitamin E als aktive Wirkstoffe (vgl. Beispiel 2) getestet.

### Ergebnisse:

An der gesunden Haut läßt sich durch das untersuchte Produkt gegenüber dem reinen Wasserbad eine temporäre, deutliche und langanhaltende Verbesserung der kapillären und epidermalen Sauerstoffversorgung um 147% bei einem Anstieg der dermalen Durchblutung um 41% erzielen.

Die Verbesserung der kutanen Sauerstoffversorgung und Durchblutung hält mit 123% bzw. 7,3% über eine Stunde nach Badeende an. Dies kann durch den sogenannten Depoteffekt des 2-Phasen-Bades erklärt werden, der eine verlängerte und intensivere Wirkung der Aktivstoffe auf die menschliche Haut ermöglicht.

Auch dieser Test belegt die hohe Effektivität der erfindungsgemäßen Zusammensetzungen durch die 2-Phasen-Wirkung (Sofort- und Depotwirkung) und der dadurch bedingten langanhaltenden Verbesserung der epidermalen Sauerstoffversorgung. Hierdurch wird die Beschaffenheit der Haut und deren Widerstandsfähigkeit sowie Aufnahmefähigkeit für Wirkstoffe nachhaltig verbessert.

Die erfindungsgemäßen Zusammensetzungen werden nach üblichen Verfahren in üblichen Apparaturen (Edelstahlkessel mit Rührwerk) hergestellt, in denen die einzelnen Komponenten nacheinander zusammengegeben, gemischt und nachfolgen konfektioniert werden. Je nach Konfektionierungsart, z.B. Ölbad oder Duschbad, können geeignete Hilfsstoffe, die hierfür üblicherweise verwendet werden, zugesetzt werden.

Die Erfindung wird anhand der nachfolgenden Beispiele näher erläutert:

### Beispiel 1

### Herstellung eines Ölbads

Sojaöl und Paraffinöl werden in einem Edelstahlkessel mit Rührer vorgelegt, unter Rühren Laureth-4 zugegeben und die nachfolgenden Komponenten in der angegebenen Reihenfolge unter Rühren zugegeben. Die Herstellung erfolgt bei einer Temperatur von 20°C bis 70°C, anschließend wird abgekühlt, filtriert (100 µm), portioniert und konfektioniert.

| Zusammensetzung 1 | |
|---|---|
| Sojaöl | 50% |
| Paraffinöl | 20% |
| Laureth-4 | 15% |
| Cocosfettsäurediethanolamid | 5% |
| Lecithin | 3% |
| Eukalyptusöl | 4% |
| Kiefernnadelöl | 2,5% |
| Tocopherolacetat | 0,5% |

### Beispiel 2

Gemäß dem Verfahren nach Beispiel 1 wurde folgende Badezusammensetzung hergestellt.

| Zusammensetzung 2 | |
|---|---|
| Sojaöl | 30% |
| Isopropylmyristat | 20% |
| Octyldodecanol | 20% |
| Jojobaöl | 5% |
| Mono/Di/Tri(alkyltetraglycolether)/o/Phosphorsäureester | 5% |
| Polyoxyethylen(7)Glycerylcocoat | 5% |
| (Lecithin) | 5% |
| Efeuextrakt | 5% |
| Panthenol | 3% |
| Parfümöl | 2% |

### Beispiel 3

Nach obigem Verfahren wurde ein Duschgel hergestellt.

| Zusammensetzung 3 | |
|---|---|
| Sojaöl | 36% |
| Paraffinöl | 20% |
| Laureth-3 | 10% |
| Polyoxyethylen(7)Glycerylcocoat | 10% |
| Capryl/Caprinsäuretriglycerid | 10% |
| Isopropylpalmitat | 8% |
| Sphingosin | 1% |
| Eukalyptusöl | 1% |
| Aerosil | 3% |
| Parfümöl | 1% |

Der Austausch von Lecithin in den obengenannten Beispielen durch andere geeignete Lipide, wie z.B. Sphingomyelin, führte zu gleich guten Ergebnissen bezüglich der vesikelbildenden Eigenschaften.

## Patentansprüche

1. Bade- und Duschzusätze mit vesikelbildenden Eigenschaften, umfassend 20 bis 90% fette Öle und/oder apolare Substanzen, dadurch gekennzeichnet, daß die Zusätze 2 bis 50% eines oder mehrerer öllöslicher, in Wasser dispergierbarer Tenside mit einem HLB-Wert von 6 bis 13 zusammen mit 0,1 bis 20% eines oder mehrerer vesikelbildender Lipide enthält.

2. Zusatz gemäß Anspruch 1, dadurch gekennzeichnet, daß er 50 bis 70%, fette Öle und/oder apolare Substanzen, 5 bis 35%, insbesondere 10 bis 20%, öllösliche Tenside und 0,5 bis 5% vesikelbildende Lipide enthält.

3. Zusatz gemäß einem der Ansprüche 1 oder 2, dadurch gekennzeichnet, daß er als fettes Öl und/oder apolare Substanz Erdnuß-, Sesam-, Sonnenblumen-, Weizenkeim-, Soja-, Jojoba-, Ricinus-, Mandel-, Nerz-, Olivenöl und/oder als apolare Substanz Paraffinöl, Isopropylmyristat, Isopropylpalmitat, mittelkettige Triglyceride, Isooctylstearat, Capryl/Caprinsäuretriglycerid, Cetylpalmitat oder deren Mischungen enthält.

4. Zusatz gemäß einem der Ansprüche 1 bis 3, dadurch gekennzeichnet, daß er als öllösliches Tensid eine öllösliche, in Wasser dispergierbare Substanz mit einem HLB-Wert von 7 bis 10 enthält.

5. Zusatz gemäß Anspruch 4, dadurch gekennzeichnet, daß der Zusatz als öllösliches Tensid Polyoxyethylenlaurylether mit 1 bis 4 EO-Einheiten, Polyoxyethylen(5)Oleylether, Polyoxyethylen(7)Glycerylcocoat, Cocosfettsäurediethanolamid, Mono/Di/Tri/(alkyltetraglycolether)/o/Phosphosäureester, Polyoxyethylen-glyceroltrioleat, Polyoxyethylensorbitantristearat und Polyoxypropylen(15)stearylether oder deren Mischungen enthält.

6. Zusatz gemäß einem der Ansprüche 1 bis 5, dadurch gekennzeichnet, daß er als vesikelbildendes Lipid gering polyoxyethylierte Fettalkohole mit einem HLB-Wert zwischen 2 und 6 und/oder Phospholipide enthält.

7. Zusatz gemäß Anspruch 6, dadurch gekennzeichnet, daß er als Fettalkohol höhere polyoxyethylierte Fettalkohole mit 1 bis 4 Ethylenoxideinheiten und als Phospholipid Lecithin, Phosphatidylcholin, -serin oder -diethanolamin oder deren Mischungen enthält.

8. Zusatz gemäß Anspruch 7, dadurch gekennzeichnet, daß der polyethoxylierte Fettalkohol von C₁₆-C₁₈-Fettalkoholen abgeleitet ist.

9. Zusatz gemäß einem der Ansprüche 1 bis 8, dadurch gekennzeichnet, daß er 0 bis 30%, vorzugsweise 5 bis 15%, wirksame Komponenten enthält.

10. Zusatz gemäß Anspruch 9, dadurch gekennzeichnet, daß er als wirksame Komponenten etherische Öle, Wirkstoffextrakte und/oder Vitamine enthält.

11. Zusatz gemäß einem der Ansprüche 1 bis 10, dadurch gekennzeichnet, daß er zusätzlich 1 bis 10 % Verdickungsmittel enthält.

12. Verfahren zur Herstellung einer Badezusammensetzung gemäß einem der Ansprüche 1 bis 11, dadurch gekennzeichnet, daß man eine Mischung aus 20 bis 90% fetten Ölen und/oder apolaren Substanzen und 0 bis 30% wirksamen Komponenten bei einer Temperatur von 20°C bis 70°C, 2 bis 50% eines oder mehrerer öllöslicher Tenside und 0,1 bis 20% eines oder mehrerer vesikelbildender Lipide zusetzt und die so gewonnene Zusammensetzung konfektioniert.

13. Verfahren gemäß Anspruch 12, dadurch gekennzeichnet, daß 50 bis 70% Ölkomponente, 5 bis 15% Wirkstoffe, 10 bis 20% Tenside und 0,5 bis 5% Lipide zugesetzt werden.

14. Verfahren gemäß Anspruch 12 oder 13, dadurch gekennzeichnet, daß der Mischung zusätzlich 1 bis 10% Verdickungsmittel zugesetzt werden.

15. Verfahren gemäß Anspruch 14, dadurch gekennzeichnet, daß als Verdickungsmittel Aerosil, Aluminium- oder Magnesiumstearat gewählt wird.

16. Verfahren gemäß einem der Ansprüche 12 bis 15, dadurch gekennzeichnet, daß als Ölkomponente Sojaöl, Paraffinöl, Iopropylmyristat, Jojobaöl, Isopropylpalmitat, Caprensäuretriglycerid oder deren Mischungen, als öllösliches Tensid Alkyltetraglycolether, Polyoxyethylen(7)Glycerolcocoat und/oder Cocosfettsäurediethanolamid und als Lipid Lecithin verwendet wird.

17. Verwendung eines Zusatzes gemäß einem der Ansprüche 1 bis 11 zur Herstellung eines Mittels für Voll-, Teil oder Duschbäder.

## Claims

1. Bath and shower additives having vesicle-forming properties, comprising 20% to 90% fatty oils and/or apolar substances, characterised in that the additives contain 2% to 50% of one or more oil-soluble surfactants dispersible in water and having an HLB value of from 6 to 13 together with 0.1% to 20% of one or more vesicle-forming lipids.

2. An additive according to claim 1, characterised in that it contains 50% to 70% fatty oils and/or apolar substances, 5% to 35%, in particular 10% to 20%, oil-soluble surfactants and 0.5% to 5% vesicle-forming lipids.

3. An additive according to either of claims 1 or 2, characterised in that it contains as fatty oil and/or apolar substance peanut oil, sesame oil, sunflower oil, wheat germ oil, soy bean oil, jojoba oil, castor oil, almond oil, mink oil, olive oil and/or as apolar substance paraffin oil, isopropyl myristate, isopropyl palmitate, medium-chain triglycerides, isooctyl stearate, caprylic/capric triglyceride, cetyl palmitate or mixtures thereof.

4. An additive according to one of claims 1 to 3, characterised in that it contains as oil-soluble surfactant an oil-soluble substance dispersible in water and having an HLB value of from 7 to 10.

5. An additive according to claim 4, characterised in that the additive contains as oil-soluble surfactant polyoxyethylene lauryl ethers having 1 to 4 EO units, polyoxyethylene(5) oleyl ether, polyoxyethylene(7) glyceryl cocoate, coconut oil fatty acid diethanolamide, mono/di/tri/(alkyltetraglycol ether)/o/phosphoric esters, polyoxyethylene glycerol trioleate, polyoxyethylene sorbitan tristearate and polyoxypropylene(15) stearyl ether or mixtures thereof.

6. An additive according to one of claims 1 to 5, characterised in that it contains as vesicle-forming lipid slightly polyoxyethylated fatty alcohols having an HLB value of between 2 and 6 and/or phospholipids.

7. An additive according to claim 6, characterised in that it contains as fatty alcohol higher polyoxyethylated fatty alcohols having 1 to 4 ethylene oxide units and as phospholipid lecithin, phosphatidyl choline, phosphatidyl serine or phosphatidyl diethanolamine or mixtures thereof.

8. An additive according to claim 7, characterised in that the polyoxyethylated fatty alcohol is derived from C₁₆-C₁₈ fatty alcohols.

9. An additive according to one of claims 1 to 8, characterised in that it contains 0% to 30%, preferably 5% to 15%, of active components.

10. An additive according to claim 9, characterised in that it contains as active components ethereal oils, extracts of active ingredients and/or vitamins.

11. An additive according to one of claims 1 to 10, characterised in that it contains in addition 1% to 10% of thickeners.

12. A process for preparing a bath composition according to one of claims 1 to 11, characterised in that to a mixture comprising 20% to 90% fatty oils and/or apolar substances and 0% to 30% active components, at a temperature of from 20°C to 70°C, 2% to 50% of one or more oil-soluble surfactants and from 0.1% to 20% of one or more vesicle-forming lipids are added and the composition thus obtained is processed.

13. A process according to claim 12, characterised in that 50% to 70% oil components, 5% to 15% active ingredients, 10% to 20% surfactants and 0.5% to 5% lipids are added.

14. A process according to claim 12 or 13, characterised in that in addition 1% to 10% of thickeners are added to the mixture.

15. A process according to claim 14, characterised in that Aerosil, aluminium stearate or magnesium stearate are selected as thickeners.

16. A process according to one of claims 12 to 15, characterised in that the oil component used is soy bean oil, paraffin oil, isopropyl myristate, jojoba oil, isopropyl palmitate, capric triglyceride or mixtures thereof, the oil-soluble surfactant used is alkyl tetraglycol ether, polyoxyethylene(7) glyceryl cocoate and/or coconut oil fatty acid diethanolamide and the lipid used is lecithin.

17. The use of an additive according to one of claims 1 to 11 for the preparation of an agent for full baths, partial baths or shower baths.

## Revendications

1. Additifs pour bain et douche ayant des propriétés de formation de vésicules, comprenant 20 à 90 % d'huiles grasses et/ou de substances apolaires, caractérisé en ce que les additifs contiennent 2 à 50 % d'un ou plusieurs tensioactifs oléosolubles dispersibles dans l'eau ayant une valeur HLB de 6 à 13, avec 0,1 à 20 % d'un ou plusieurs lipides formant des vésicules.

2. Additif selon la revendication 1, caractérise, en ce qu'il contient 50 à 70 % d'huiles grasses et/ou de substances apolaires, 5 à 35 %, notamment 10 à 20 %, de tensioactifs oléosolubles et 0,5 à 5 % de lipides formant des vésicules.

3. Additif selon une des revendications 1 ou 2, caractérisé en ce qu'il contient comme huile grasse et/ou substances apolaires de l'huile d'arachide, de l'huile de sésame, de l'huile de tournesol, de l'huile de germes de blé, de l'huile de soja, de l'huile de jojoba, de l'huile de ricin, de l'huile d'amande, de l'huile de vison, de l'huile d'olives et/ou comme substance apolaire de l'huile de paraffine, du myristate d'isopropyle, du palmitate d'isopropyle, des triglycérides à chaîne moyenne, du stéarate d'isooctyle, du triglycéride d'acide caprique/caprylique, du palmitate de cétyle ou leurs mélanges.

4. Additif selon l'une des revendications 1 à 3, caractérisé en ce qu'il contient comme tensioactif oléosoluble une substance oléosoluble dispersible dans l'eau ayant une valeur HLB de 7 à 10.

5. Additif selon la revendication 4, caractérisé en ce que l'additif contient comme tensioactif oléosoluble de l'éther laurylique de polyoxyéthylène ayant 1 à 4 motifs EO, de l'éther oléylique de polyoxyéthylène(5), du cocoate de glycéryle de polyoxyéthylène(7), du diéthanolamide d'acide gras de coco, du mono/di/tri/(éther d'alkyltétraglycol)/ester o-phosphorique, du trioléate de glycérol-polyoxyéthylène, du tristéarate de sorbitane-polyoxyéthylène et de l'éther stéarylique de polyoxypropylène(15) ou leurs mélanges.

6. Additif selon l'une quelconque des revendications 1 à 5, caractérisé en ce qu'il contient comme lipide formant des vésicules des alcools gras peu polyoxyéthylés ayant une valeur HLB comprise entre 2 et 6 et/ou des phospholipides.

7. Additif selon la revendication 6, caractérisé en ce qu'il contient comme alcool gras des alcools gras supérieurs polyoxyéthylés ayant 1 à 4 motifs oxyde d'éthylène et comme phospholipide de la lécithine, de la phosphatidylcholine, de la phosphatidylsérine ou de la phosphatidyldiéthanolamine ou leurs mélanges.

8. Additif selon la revendication 7, caractérisé en ce que l'alcool gras polyoxyéthylé est dérivé d'alcools gras en C₁₆-C₁₈.

9. Additif selon l'une des revendications 1 à 8, caractérisé en ce qu'il contient 0 à 30 %, de préférence 5 à 15 %, de composants actifs.

10. Additif selon la revendication 9, caractérisé en ce qu'il contient comme composants actifs des huiles essentielles, des extraits de principe actif et/ou des vitamines.

11. Additif selon une des revendications 1 à 10, caractérisé en ce qu'il contient en outre 1 à 10 % d'un épaississant.

12. Procédé de production d'une composition de bain selon une des revendications 1 à 11, caractérisé en ce qu'on ajoute à un mélange composé de 20 à 90 % d'huiles grasses et/ou de substances apolaires et de 0 à 30 % de composants actifs à une température de 20°C à 70°C, 2 à 50 % d'un ou plusieurs tensioactifs oléosolubles et 0,1 à 20 % d'un ou plusieurs lipides formant des vésicules et qu'on prépare industriellement la composition ainsi obtenue.

13. Procédé selon la revendication 12, caractérisé en ce qu'on ajoute 50 à 70 % de composants huileux, 5 à 15 % de principes actifs, 10 à 20 % de tensioactifs et 0,5 à 5 % de lipides.

14. Procédé selon la revendication 12 ou 13, caractérisé en ce qu'on ajoute additionnellement au mélange 1 à 10 % d'épaississant.

15. Procédé selon la revendication 14, caractérisé en ce qu'on choisit comme épaississant de l'Aérosil, du stéarate d'aluminium ou du stéarate de magnésium.

16. Procédé selon une des revendications 12 à 15, caractérisé en ce qu'on utilise comme composants huileux de l'huile de soja, de l'huile de paraffine, du myristate d'isopropyle, de l'huile de jojoba, du palmitate d'isopropyle, du triglycéride d'acide caprénique ou leurs mélanges, comme tensioactif oléosoluble de l'éther d'alkyltétraglycol, du cocoate de glycérol-polyoxyéthylène(7) et/ou du diéthanolamide d'acide gras de coco et comme lipide de la lécithine.

17. Utilisation d'un additif selon une des revendications 1 à 11 pour la production d'un agent pour bain complet, bain partiel ou bain et douche.
